# EUROPEAN PATENT SPECIFICATION

(11) **EP 3 903 662 B1**
(45) Date of publication and mention of the grant of the patent: **14.10.2026**
(21) Application number: 20172242.8
(22) Date of filing: 30.04.2020
(51) Int. Cl.: A61B 1/00, A61B 1/005

(54) **AN ENDOSCOPE CONTROL SYSTEM**
ENDOSKOPSTEUERUNGSSYSTEM
SYSTÈME DE COMMANDE D'ENDOSCOPE

(43) Date of publication of application: 03.11.2021
(73) Proprietor: Ambu A/S, 2750 Ballerup (DK)
(72) Inventor: NIELSEN, Martin Refslund, 3460 Birkerød (DK)
(74) Representative: AWA Denmark A/S

(56) References cited:
- JP-A- H0 938 028
- US-A1- 2012 277 535
- US-A1- 2014 343 489

## Description

The present disclosure relates to insertable medical vision devices, such as, but not limited to, endoscopes, in particular disposable insertion endoscopes, such as duodenoscopes, gastroscopes, and colonoscopes. More specifically, the present disclosure relates to endoscope control systems comprising control wheels connected to associated wire drums for connection to steering wires, whereby rotation of the control wheels controls a bending operation of a tip of the endoscope, and wherein activation of one or more brakes brake rotation of one or more control wheels.

Endoscopes are typically equipped with a light source and a vision receptor including a vision or image sensor. Provided that enough light is present, it is possible for the operator to see where the endoscope is steered and to set the target of interest once the tip has been advanced thereto.

Endoscopes typically comprise an elongated insertion tube with a handle at the proximal end, as seen from the operator, and visual inspection means, such as a built-in camera including a vision sensor, at a distal end of the elongated insertion tube. This definition of the terms distal and proximal, i.e. "proximal" being the end closest to the operator and "distal" being the end remote from the operator, as used herein for endoscopes in general, is adhered to in the present specification. Electrical wiring for the camera and other electronics, such as one or more LEDs accommodated in the tip part at the distal end, runs along the inside of the elongated insertion tube from the handle to the tip part. A working or suction channel may run along the inside of the insertion tube from the handle to the tip part, e.g. allowing liquid to be removed from the body cavity or allowing for insertion of surgical instruments or the like into the body cavity. The suction channel may be connected to a suction connector, typically positioned at a handle at the proximal end of the insertion tube.

To be able to maneuver the endoscope inside the body cavity, the distal end of some endoscopes comprises a bendable distal tip, which may be bendable in one, e.g. an up/down dimension, or two dimensions, e.g. an up/down dimension and a left/right dimension. The bendable tip often comprises a bending section with increased flexibility, e.g. achieved by articulated segments of the bending section. The maneuvering of the endoscope inside the body is typically done by tensioning or slacking steering wires also running along the inside of the elongated insertion tube from the tip part through the remainder of articulated segments to a control system or control mechanism positioned in or forming part of the handle.

An endoscope control system for performing a bending operation in two dimensions is known from WO2018022418A2. This control system includes two control wheels connected two associated wire drums for connection to associated steering wires of the endoscope, whereby rotation of the control wheels controls the bending operation in two dimensions. The control system includes a brake activated by a brake handle.

US9,949,623B discloses another endoscope control system including a control wheel and a brake.

Multi-disc brakes, also known as multiple disc brakes, multi-plate brakes, or multiple plate brakes, are well-known in technical fields far removed from that of endoscopes, e.g. in heavy machinery in the pressing industry and winching machinery. Technically, these types of brakes are closely related to multi-disc clutches, also known as multiple disc clutches, multi-plate clutches, or multiple-plate clutches. Other well-known applications of multi-disc brakes include in machinery for agriculture, motorcycles, and race cars.

Applying generally to this disclosure, the term "comprises" includes "consists essentially of". In this disclosure, the term "to accommodate" may additionally or alternatively be defined as "to house" or "to enclose" or "to surround".

In this specification, the terms "integrally" or "integrally provided" or "integrally comprising", "in one piece" or similar may be defined as the associated features forming an integral part of a whole; and/or are in one piece, potentially molded in one piece; and/or are substantially inseparable by hand.

As mentioned, in this specification, the term "proximal" may be defined as being closest to an operator of the endoscope, and the term "distal" as being remote from the operator. The term "proximal-distal" may be defined as extending between these two extremes, in the present case proximal-distal may extend along a center axis of the tip part extending between a proximal extremity of the proximal end of the tip part and a distal extremity of the distal end of the tip part.

In this specification, an endoscope may be defined as a device adapted for viewing body cavities and/or channels of a human and/or animal body. The endoscope may for instance be a flexible or steerable endoscope. The endoscope may be a duodenoscope or a ureteroscope, a gastroscope, or a colonoscope.

A first aspect of this disclosure, according to the invention, relates to an endoscope control system for performing a bending operation in a disposable insertion endoscope, the endoscope control system comprising:
a control wheel connected to a wire drum for connection to a steering wire of the endoscope, whereby rotation of the control wheel controls the bending operation; and
a multi-disc brake comprising a stack of at least three brake discs, wherein activation of the multi-disc brake changes the multi-disc brake from a released state to a braking state, a brake torque generated by the multi-disc brake in the braking state braking rotation of the control wheel, the brake torque in the released state being at least partially released.

An endoscope control system comprising a multi-disc brake is e.g. known from US 2012/277535 A1 which however does not does not use wires and wire drums for performing the bending operation, but chains and sprockets.

Other endoscope control systems comprising a brake are e.g. known from US 2014/343489 A1 and JP H09 38028 A.

According to the invention, as claimed in claim 1, the control system further comprises a spring exerting a spring force on the stack of brake discs in the engaged and activated state, i.e. in the braking state of the multi-disc brake as a means for activating the braking function of the stack of brake discs, and the stack of brake discs consists of four or more discs.

With such a multi-disc brake, a relatively high braking torque may be achievable with relatively small dimensions, such as a diameter, of the brake. Load and friction may be shared between several and potentially larger friction interfaces, and a more consistent and predictable brake torque may be achievable.

Furthermore, such a multi-disc brake may make it possible to better control the braking torque (in the braking state), which should be high enough to leave a tip of the endoscope in a desired bent or unbent position, but not so high as to risk injury to or damaged tissue of a patient, e.g. if the physician by mistake should attempt to retract the tip from the patient without releasing the brake. In this case the tip must be allowed to move before any injuries to the tissue can take place. If the control system includes a spring as described further below, a smaller spring and/or a spring of lower spring constant may potentially be suitable, and tolerances of the spring force may be improved. A longer pre-loaded spring with a low spring constant may achieve a robust design and a lower long-term mechanical stress on surrounding elements, which may make it possible to manufacture such elements from materials of lower strength and/or rigidity, such as plastic polymers. See also further below.

Furthermore, such a multi-disc brake may be made compact and/or easy to engage and/or disengage and/or may be manufacturable with low cost while being able to withstand forces as required by the intended use and/or may be provided so that it achieves a consistent brake torque when engaged.

Furthermore, such a multi-disc brake may achieve a predictable and consistent brake torque. This may be advantageous in some applications where the brake torque should be within a specific range.

The multi-disc brake may be a friction brake which may work by friction members sliding on each other at a certain diameter across a rotation axis. One friction member may be attached to a frame and one friction member may be attached to a body of which movement is to be braked. A normal force may act on pairs of friction members, potentially resulting in a friction force working against movement at a contact diameter. This may result in a brake torque proportional with the normal force, contact diameter and a coefficient of friction.

In order to achieve a consistent brake torque within a specific range, pairs of friction members with consistent coefficient of friction can be applied. Friction members with a relatively consistent coefficient of friction (low variance of the coefficient of friction) typically have a relatively low coefficient of friction. Consistent and low coefficients of friction can be achieved with plastics materials, which may provide the possibility of using low cost injection molded components.

The pairs of friction members may slide at a limited diameter; hence, the brake may be compact in order to fit into a handle assembly of the endoscope. In a conventional friction brake, a combination of a low coefficient of friction and a limited working diameter may result in a need for a high normal force. A high normal force may not be desirable; hence, the brake may be dimensioned to be stronger and more rigid, which may compromise a desire to make a cheaper brake and a desire to make it easy to engage and disengage the brake. Multiple friction surfaces working in parallel may eliminate a need of a high normal force even though the coefficient of friction and the diameter are limited. Hence, the brake torque is proportional with the number of friction interfaces working in parallel. Multiple friction interfaces can be made in a simple way by stacking friction discs on top of each other. Every other disc may be rotationally attached to the frame, and the remaining discs in between may be attached to the body which is intended to be braked. A compression force on the disc stack may result in a similar normal force between each pair friction of friction members. Hence, a limited compression force on the stack may provide a sufficient brake torque (even though the coefficient of friction and the diameter are limited). This may limit the structural requirements of the brake components. A further advantage of having multiple sliding surfaces may be that heat generation and wear may be distributed between multiple interfaces. Reduced component requirements for structure, wear, heat resistance, and heat transfer may make it possible to use low cost, injection molded plastic parts.

It may be desired that the compression force on the disc stack is within a specific range for the brake torque to be within a specific range. By using a pre-compressed spring which is contacted and compressed a little further when engaging the brake, a relatively precise total spring compression may be achieved. Component tolerances (including a tolerance of a free length of the spring) may be relatively small in relation to the total compression of the spring. This may result in a consistent spring force and a robust brake system.

The endoscope control system can alternatively be denoted an endoscope bending operation apparatus.

The control system may be positioned on or in, or may form part, of an endoscope handle of the endoscope, see also further below.

One or more or all the brake discs may be rings circumscribing an axis of rotation of the control wheel. The rings may have a center opening through which a center shaft of the control system and/or other components, such as the spring may extend. The center opening may be relatively large compared to a diameter of the rings.

One or more of the first to third brake discs or further brake discs may be shaped as rings, i.e. as discs with a relatively small center opening.

One or more of the first to third brake discs or further brake discs may be split into disc or ring segments, such as two, three, or more segments. However, in this case, every second brake disc in the stack may not be split into such segments, i.e. may form a full ring. This may provide a multi-disc brake, where the disc segments or ring segments could be considered to form brake shoes acting on an adjacent, full-ring brake disc.

The control system may comprise a brake handle or a similar activation device or activation means, movement of which changes the multi-disc brake between the braking state and the released state. Such movement may be a rotation, potentially about a rotation axis of the control wheel. Movement of the brake handle may be transferred to the multi-disc brake by the brake handle rotating a rotation member, such as a disc, relative to a sliding member, such as a disc or one of the brake discs, the sliding member providing a pushing force on at least one of the brake discs activating the braking torque on the multi-disc brake. The rotation member and/or the sliding member may include an inclined portion or ramp so that rotating movement between the member along the inclined portion pushes the two members away from each other.

The brake handle may be positioned so that the brake handle does not touch the control wheel during the movement of the brake handle. The brake handle may comprise an arm that extends to the multi-disc brake.

Each of the brake discs may be formed in one piece or may include or be assembled from several pieces.

Every other of the brake discs of the stack may be connected or fixed to each other, and/or the remaining brake discs may similarly be connected or fixed to each other, these two sets of brake discs potentially being rotatable in relation to each other.

In the released state of the multi-disc brake, the springs(s) described further below may relieve or release frictional engagement of the brake discs and/or may push the brake discs out of frictional engagement.

All parts of the control system, potentially except for the spring(s) described below and/or steering wires and/or one or more of the brake discs may be manufactured from plastic polymer(s).

In some embodiments, the stack includes a first brake disc positioned between a second and a third brake disc of the stack, the first brake disc being rotatable in relation to the second and third brake discs, a first friction interface being provided between the first and second discs and a second friction interface being provided between the first and third brake discs, so that rotation of the first brake disc in relation to the second and third brake discs activates the first and second friction interfaces to provide at least part of the brake torque.

In embodiments where the control system comprises more than three, such as six or nine, brake discs, the entire stack or part of the stack of brake discs may be provided in a similar manner, i.e. so that such friction interfaces are provided between adjacent brake discs.

In some embodiments of the control system, a first of the brake discs is positioned between a second and a third of the brake discs so that the first brake disc has a first friction interface with the second brake disc and a second friction interface with the third brake disc, the first and second friction interfaces being activated in the braking state of the multi-disc brake and being at least partly released or deactivated in the released state of the multi-disc brake.

A set of discs, including e.g. the first third discs, may be stationary discs, and a set of discs, including e.g. the second disc, positioned in between may be rotating discs. The set of stationary discs may be fixed in relation to the frame or handle housing of the endoscope as described elsewhere herein. The set of rotating discs may be fixed in relation to a rotating part of the control system including the control wheel. These two different types of discs can be placed in any order, preferably alternating. Friction interfaces may be present where the two different types of brake discs interface.

The friction interfaces may include at least parts of two opposed major surfaces of the first brake disc, these major surfaces facing or abutting corresponding major surfaces of the second and third brake discs, the friction interfaces similarly potentially including at least part of these second and third brake disc major surfaces. If the stack includes more than three brake discs, the further brake discs may be arranged in a corresponding manner.

In a further development of the control system, when moving the multi disc brake from the released state to the braking state, a force is exerted on the stack of discs, the force pushing the second and third discs towards the first disc, the first and second friction interfaces thereby providing a brake force against rotation of the first brake disc relative to the second and third brake discs.

In another or further development, the control system further comprises a frame, the rotation of the control wheel occurring relative to the frame, and wherein the first brake disc is rotationally fixed relative to the control wheel, and the second and third brake discs are rotationally fixed relative to the frame.

The control wheel may be rotatable in relation to the frame. In embodiments where the control system comprises more than three brake discs, the brake discs may generally be arranged in the stack so that every other brake disc is rotationally fixed relative to the control wheel, and the remaining brake discs are rotationally fixed relative to the frame.

In a development of the latter embodiments of the control system, the frame is fixed to or forms part of an endoscope handle housing of an endoscope handle.

The handle housing may be a handle shell.

The frame and/or the housing may be manufactured of a rigid material, such as a rigid plastic polymer.

In some embodiments of the control system, the stack consists of six brake discs.

The stack may consist of five or more, six or more, seven or more, eight or more, nine or more, or ten or more brake discs.

In some embodiments of the control system, every other of the brake discs of the stack is manufactured from a material different from a material of the remaining brake discs.

Hereby, friction properties in the friction interfaces between associated or abutting brake discs may be controlled. In particular, by selecting suitable different materials, static friction may be lowered, which may improve control. For example, one material, such as polycarbonate (PC) or polypropylene (PP) may be selected for a first set of brake discs, and another material, such as acrylonitrile butadiene styrene (ABS), for the other set.

In some embodiments of the control system, at least one of the brake discs is manufactured from plastic polymer material.

Two or three or more or all brake discs may be manufactured from such material.

Especially if all brake discs are of such material, this may make the control system particularly suitable for disposable or one-use endoscopes.

The plastic polymer material may comprise or consist of one or more of PC, PP, ABS, polyethylene (PE), polyamide (PA), polyurethane (PU), polystyrene (PS), polylactic acid (PLA), polyvinyl chloride (PVC), polyoxymethylene (POM), polyester, polyethylene terephthalate (PET), and acrylic (PMMA). The polymer may be a copolymer of one or more monomers of the latter materials.

One or more of the brake discs may alternatively or additionally comprise or consist of a metal, such as steel, which may provide higher thermal conductivity. One of the abovementioned sets of brake discs may be of the plastic polymer material, and the other set of the metal.

Some embodiments of the control system further comprise a helical compression spring exerting a spring force on the stack of brake discs in the engaged state of the multi-disc brake.

The helical spring may be positioned coaxially with a center axis or center shaft of the control system or of the control wheel.

The helical spring may comprise or consist of metal, e.g. steel.

A spring characteristic of the helical spring can be made linear or substantially linear.

As alternatives to a helical spring, any other suitable spring may be used. The spring may be a diaphragm spring, cup spring, disc spring, saucer spring, or leaf spring. The spring or a spring function may be provided by or integrated in another component, such one of the brake discs, e.g. the first brake disc, or one set of brake discs being of a resilient material, such as a resin or a spring steel, and other discs or the other set of brake discs being of a substantially non-resilient material, such as (non-spring) steel. Alternatively, the spring is provided separately from the brake discs.

As alternatives to a compression spring, the spring may be a tension spring or a drive spring.

The control system may include two or more springs (for each control wheel, see further below), which may be helical and/or compression springs, or a combination of the above spring types may be used.

Instead of a spring, other means for activating the braking function of the stack of brake discs may be provided. Such other means could include an element, which can move a brake disc positioned at an end of the stack towards the other brake discs of the stack to provide the brake torque without providing a spring function. Such an element could be activated by a brake handle.

In a development of these embodiments, the spring is prestressed.

Advantages of a precompressed or prestressed spring may include that such a spring can provide a higher brake torque at smaller movements and/or that precise control can be provided at smaller movements. The necessary mechanical pressure can thus be achieved with a relatively limited movement of a brake handle. An advantage of such spring may be that compression of the spring in relation to a free length of the spring can be very precisely controlled by a small additional compression, resulting in a consistent spring force, thereby providing a more robust brake system. A small compression may require a small amount of energy, making it possible to engage the brake more easily by means of a brake handle.

In some embodiments, the control system further comprises:
a further control wheel connected to a further wire drum for connection to a further steering wire of the endoscope, whereby rotation of the further control wheel controls the bending operation in another dimension than that of the control wheel; and
a further multi-disc brake comprising a stack of at least three brake discs, wherein activation of the further multi-disc brake changes the further multi-disc brake from a released state to a braking state, a brake torque generated by the further multi-disc brake in the braking state braking rotation of the further control wheel, the brake torque in the released state being at least partially released.

The further control wheel and/or wire drum and/or steering wire and/or multi-disc brake may be embodied in accordance with any one of the above embodiments of the (first) control wheel, wire drum, steering wire, and multi-disc brake, respectively.

The further control wheel may be positioned coaxially with and potentially axially shifted in relation to the (first) control wheel. A diameter or a cross-sectional dimension of the two control wheels may be different from each other, potentially so that an outer one of the two control wheels has a smaller diameter or smaller cross-sectional dimension.

Alternatively, the further multi-disc brake may be replaced with a brake of another type.

In another aspect, the present disclosure involves an endoscope handle for an endoscope, the endoscope handle comprising a control system according to any one of above embodiments.

In another aspect, the present disclosure involves an endoscope comprising a control system according to any one of the above embodiments and/or an endoscope according to any one of the above embodiments.

The endoscope may further comprise an endoscope handle at the proximal end thereof, and/or visual inspection means, such as a built-in camera including a vision sensor, at a distal tip. Electrical wiring for the camera and other electronics, such as one or more LEDs accommodated in the tip part at the distal end, may run along the inside of the elongated insertion tube from the endoscope handle to a PCB or an FPC at the distal tip. A working or suction channel may run along the inside of the insertion tube from the handle to the tip part, e.g. allowing liquid to be removed from the body cavity or allowing for insertion of surgical and/or sampling instruments or the like into the body cavity. The suction channel may be connected to a suction connector, typically positioned at a handle at the proximal end of the insertion tube.

In some embodiments of the endoscope, the endoscope further comprises a distal tip or tip part that comprises a bending section connected to the steering wire(s) so that the control system can activate a bending operation of the bending section via the steering wire(s).

The bending section may be bendable in one or two dimensions, e.g. an up/down dimension and a left/right dimension. The bendable tip may comprise a bending section with increased flexibility, e.g. achieved by articulated segments of the bending section as are known in the art. The steering wire(s) may run along the inside of an elongated insertion tube from the tip through the bending section to the control system positioned in or forming part of the endoscope handle.

The endoscope may be a disposable insertion endoscope. The endoscope may include one or more features as described herein in the above, including the features of endoscopes described in the above introduction to this description, and in connection with the description of the methods and tip parts according to the present disclosure.

The endoscope may comprise an elongated insertion tube with a handle at the proximal end. A tip or tip part may be positioned at the distal end of the elongated insertion tube. The tip may further comprise a bending section positioned between the tip and the elongated insertion tube. The bending section may be configured to be articulated to maneuver the endoscope inside a body cavity.

The endoscope may be a duodenoscope, a gastroscope, or a colonoscope.

A person skilled in the art will appreciate that any one or more of the above aspects of this disclosure and embodiments thereof may be combined with any one or more of the other aspects and embodiments thereof.

In the following, non-limiting exemplary embodiments will be described in greater detail with reference to the drawings, in which:
Fig. 1 shows a perspective view of an embodiment of the endoscopes according to the present disclosure, the endoscope including an embodiment of the control systems according to the present disclosure;
Fig. 2 shows a top view of the control system of Fig. 1;
Fig. 3 shows a cross section taken along line A-A in Fig. 2;
Fig. 4 shows a perspective view of a handle of the endoscope of Fig. 1, a first multi-disc brake of a first control wheel of the control system being shown in an exploded view;
Fig. 5 shows a perspective view of a detail of the control system of Fig. 2 with some parts removed and parts of the brake of Fig. 4 shown in an assembled state; and
Fig. 6 shows an exploded perspective view of parts of the endoscope of Fig. 1, including of a second multi-disc brake of a second control wheel.

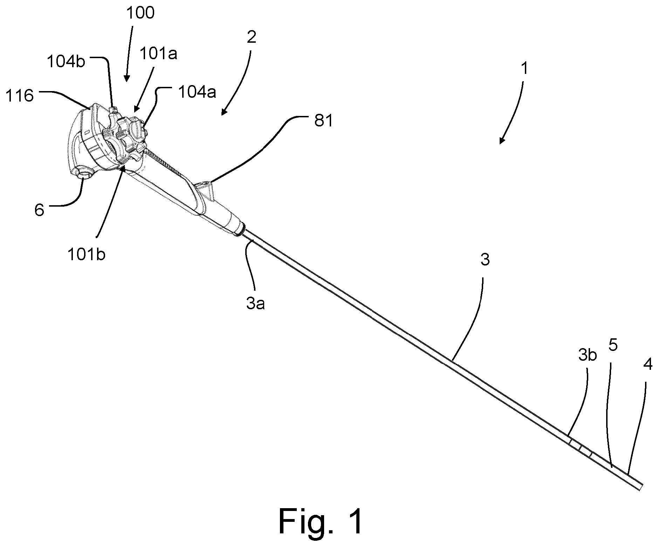

Fig. 1 shows a perspective view of a disposable insertion endoscope 1 with a control system 100, an elongated insertion tube 3, and an endoscope handle 2 at a proximal end 3a of the elongated insertion tube 3. In a known manner, an endoscope tip 4 is positioned at a distal end 3b of the elongated insertion tube 3, the tip 4 comprising a bending section 5 positioned between the tip 4 and the elongated insertion tube 3. The endoscope handle 2 comprises the endoscope control system 100, the endoscope control system 100 being for performing a bending operation of the disposable insertion endoscope 1.

In a known manner, the bending section 5 is connected to (not shown) steering wires, which extend from the control system 100 through the tube 3 to allow the control system 100 to activate a two-dimensional bending operation of the bending section 5 via the steering wires. The bending section 5 is configured to be articulated to maneuver the endoscope 1 inside a body cavity (not shown). The bending section 5 is bendable in two dimensions, i.e. an up/down dimension and a left/right dimension. In an alternative, not shown embodiment, the bending section is bendable in one dimension only. The bending section 5 has increased flexibility achieved by articulated segments of the bending section 5 as is known in the art. The steering wires run along the inside of the elongated insertion tube 3 from the tip 4 through the bending section 5 to the endoscope control system 100. Still in a known manner, the maneuvering of the endoscope 1 inside the body can be carried out by tensioning or slacking the steering wires by means of the control system 100.

Still in a known manner, the distal tip 4 has a not shown built-in camera including a vision sensor. Not shown electrical wiring for the camera and potential other electronics, such as one or more LEDs accommodated in the tip part 4, run along the inside of the elongated insertion tube 3 from the endoscope handle 2 to a PCB or an FPC at or in the distal tip 4. A not shown suction/working channel runs along the inside of the insertion tube 3 from the handle 2 to the tip part 4, e.g. allowing liquid to be removed from the body cavity or allowing for insertion of a surgical instrument and/or a sampling instrument or other instruments (not shown) into the body cavity. The suction channel is connected to a suction connector 6 positioned at the handle 2 at the proximal end 3a of the insertion tube 3.

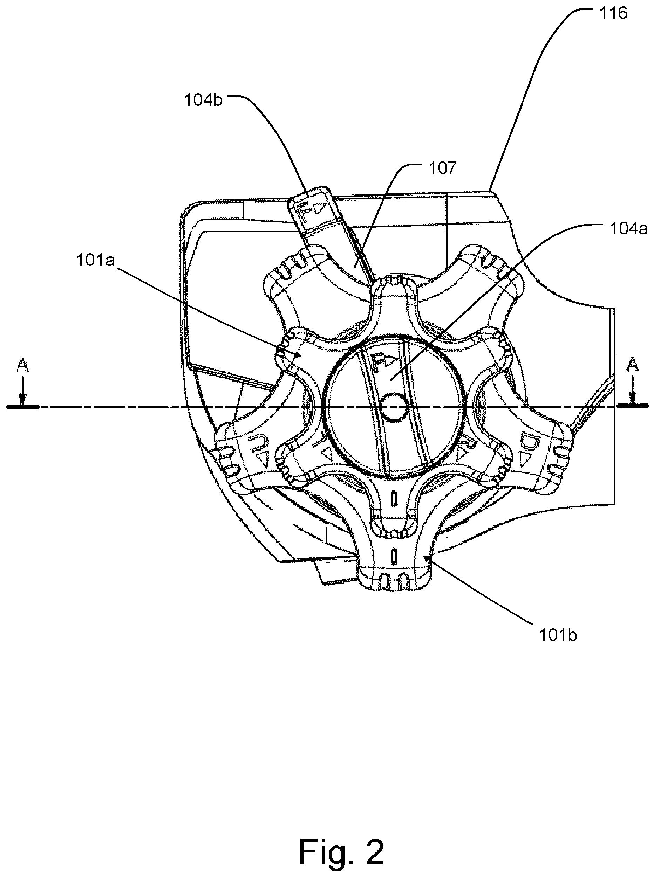

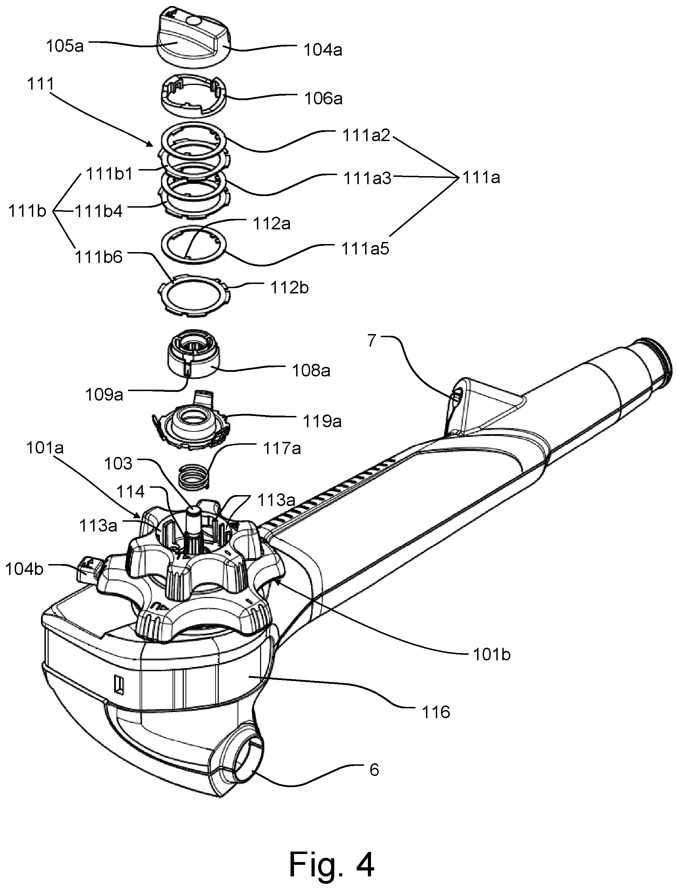

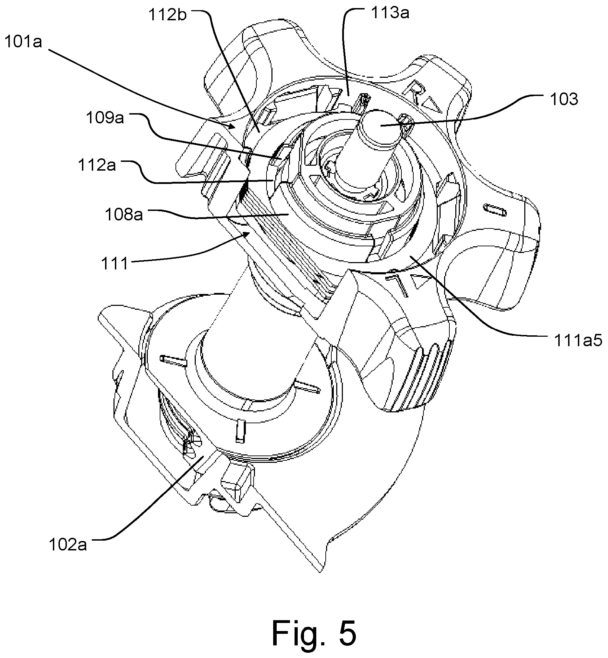

Referring to Figs 1 to 6, the control system 100 comprises a first control wheel 101a connected to a wire drum 102a for connection to a steering wire of the endoscope 1, whereby rotation of the control wheel 101a controls the bending operation by rotating the wire drum 102a to, in a known manner, to activate the not shown steering wire, the steering wire being connected to the wire drum 102a. The control system 100 further comprises a first multi-disc brake 110a comprising a stack 111 of two brake disc sets 111a, 111b , see e.g. Fig. 4. Activation of the brake 110 changes the multi-disc brake 110 from a released state to a braking state. In the braking state, a brake torque generated by the brake 100 brakes rotation of the control wheel 101a, the brake torque being released in the released state. The brake 110 makes it possible to control the braking torque (in the braking state). The brake 110 is designed so that the braking torque is high enough to leave the tip 4 of the endoscope 1 in a desired bent or unbent position, but not so high as to risk injury to or damaged tissue of a patient, e.g. if the physician by mistake should attempt to retract the tip 4 from the patient without releasing the brake 110.

The brake discs shown at 111a and 111b are shaped as rings circumscribing an axis of rotation of the control wheel 101a. The ring-shaped brake discs shown at 111a and 111b have a center opening through which a center shaft 103 of the control system 100 extends. The center opening is of relatively large diameter compared to a total diameter of the rings.

The control system 100 further comprises an activation device in the form of a brake knob 104a, rotation of the brake knob 104a about a rotation axis of the control wheel 101a moving the brake 110 between the braking state and the released state. Movement of the brake knob 104a rotates a disc-shaped rotation member 105a thereof relative to a disc-shaped sliding member 106a. In the braking state, the sliding member 106a activates an axial force pushing the brake discs at 111a, 111b towards each other to activate the braking torque of the brake 110. The rotation member 105a and the sliding member 106a include interacting inclined portions so that mutual rotation along the inclined portions in a per se known manner pushes the rotation member 105a and the sliding member 106a away from each other, i.e. the sliding member 106a is pushed towards the brake 110 to activate the break 110 as will be described further below.

As best seen in Fig. 4, each of the brake discs at 111a, 111b is formed in one piece, every other of the brake discs, i.e. the brake discs at 111a, are connected to each other by a disc holder 108a, where disc protrusions 112a engage disc holder grooves 109a. The remaining brake discs at 111b are similarly connected to each other by the control wheel 101a, disc protrusions 112b of the brake discs 111b engaging control wheel grooves 113a, so that the two sets 111a, 111b of brake discs are rotatable in relation to each other.

The set 111b includes a first brake disc 111b1 positioned between a second and a third brake disc 111a2, 111a3 of the set 111a, the first brake disc 111b1 being rotatable in relation to the second and third brake discs 111a2, 111a3, a first friction interface being provided between the first and second brake discs 111b1, 111a2, and a second friction interface being provided between the first and third brake discs 111b1, 111a3 so that rotation of the first brake disc 111b1 in relation to the second and third brake discs 111a2, 111a3 activates the first and second friction interfaces to provide part of the brake torque.

The first brake disc 111b1 being rotatable in relation to the second brake disc 111a2 and the third brake disc 111a3 is realized by the first brake disc 111b1 being attached to the control wheel 101a by the control wheel grooves 113a and the second and third brake discs 111a2, 111a3 being attached to the disc holder 108a by the disc holder grooves 109a as described above. The disc holder 108a is attached to the center shaft 103 via the center shaft grooves 114, the center shaft 103 being fixed in the handle 2 and being rotatably fixed relative to the handle 2.

In the embodiment shown, the control system 100 comprises a total of six brake discs distributed with three discs in each of the sets 111a, 111b, where the entire stack of brake discs 111 is provided in a similar manner, i.e. so that such friction interfaces are provided between adjacent brake discs, and the first set of brake discs 111a being provided similarly to the second and third brake discs 111a2 and 111a3, and the brake discs of the second set 111b being provided similarly to 111b1. That is, the brake discs are arranged in the stack 111 so that every other brake disc i.e. brake discs 111a, are fixed to and rotate with the control wheel 101a, and the remaining brake discs, i.e. brake discs 111b, are fixed to the disc holder 108a which is non-rotatably fixed to the center shaft 103. As the center shaft 103 is non-rotatably fixed to the handle 2, the control wheel 101a and connected brake discs 111a may be rotated relative to the center shaft 103 (and handle) to rotate the wire drum 102a and control the bending operation of the endoscope 1. To guide and facilitate rotation of the wire drum 102a and control wheel 101a, the wire drum 102a and control wheel 101a are journaled in an inner bearing member 120a. In other embodiments, alternative suitable bearing devices may be applied. The stack 111 may alternatively consist of three, four, five, seven, eight, nine, ten, or more brake discs.

The first of the brake discs 111b1 is positioned between the second and third brake discs 111a2, 111a3 so that the first and second friction interfaces are activated in the braking state of the multi-disc brake 110 and are at least partly released or deactivated in the released state of the multi-disc brake 110. The friction interfaces include parts of two opposed major surfaces of the first brake disc 111b1, these major surfaces facing corresponding major surfaces of the second and third brake discs 111a2, 111a3, respectively, the friction interfaces similarly including parts of the associated second and third brake disc major surfaces.

Remaining brake discs 111b4, 111b6, and 111a5 of the stack 111 are arranged in a corresponding manner. Thus, the brake disc 111b4 is positioned between the brake discs 111a3, 111a5 so that corresponding friction interfaces are correspondingly and simultaneously activated in the braking state of the multi-disc brake 110 and are at least partly released or deactivated in the released state of the multi-disc brake 110. These friction interfaces correspondingly include parts of two opposed major surfaces of the brake disc 111b4, these major surfaces facing corresponding major surfaces of the brake discs 111a3, 111b2, the friction interfaces similarly including parts of the associated brake disc major surfaces.

When moving the multi-disc brake 110 from the released state to the braking state by rotation of the knob 104a, a force is exerted on the stack of discs 111 by the sliding member 106a, the force pushing the second and third discs 111a2, 111a3 towards the first disc 111b1, the first and second friction interfaces thereby providing the brake force against rotation of the first brake disc 111b1 relative to the second and third brake discs 111a2, 111a3. Similar action occurs throughout the rest of the stack 111.

The center shaft comprises a frame 115 fixed to the endoscope handle 2 by means of screws (not shown) and via the inner bearing element 120a, the rotation of the control wheel 101a occurring relative to the frame 115. The first brake disc 111b1 is rotationally fixed relative to the control wheel 101a, and the second and third brake discs 111a2, 111a3 are rotationally fixed relative to the frame 115.

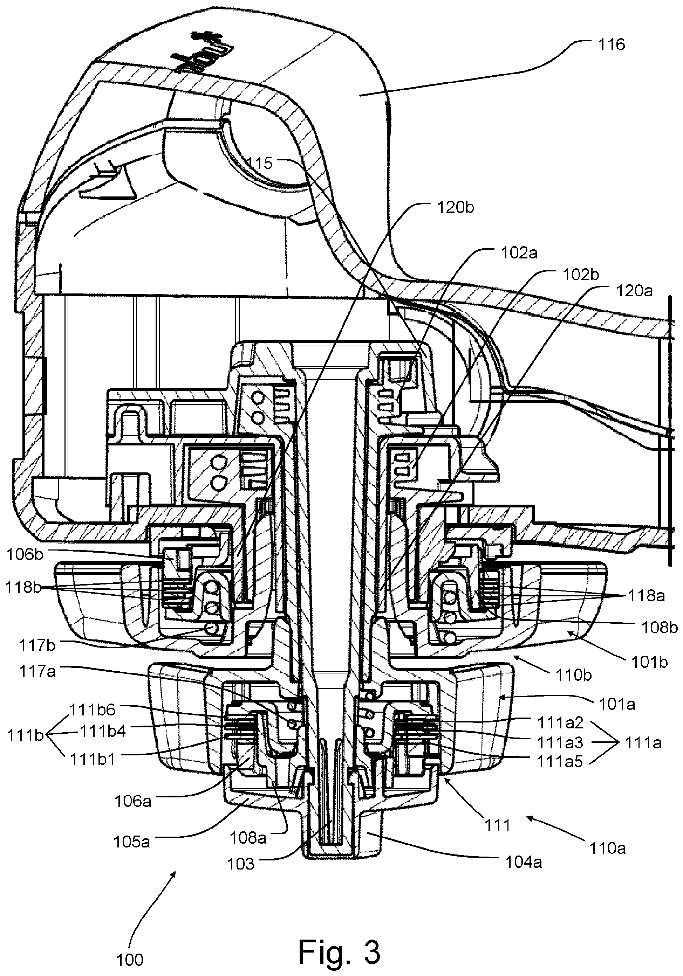

As best seen in Fig. 3, the handle housing 116 forms a handle shell encasing parts of the control system 100. Both the frame 115 and the housing 116 are manufactured of a rigid plastic polymer in the form of ABS.

The brake discs of each set 111a, 111b are manufactured from different materials, i.e. contiguous brake discs are of different materials. Hereby, friction properties of the friction interfaces between adjacent brake discs is controlled. In particular, by selecting suitable different materials, static friction is lowered, improving control of the bending operation. PC is selected for the first set 111a, and ABS for the other set of brake discs 111b. The brake discs of the sets 111a, 111b may alternatively be made from other plastic polymer materials as disclosed above. In other embodiments, one or more of the brake discs of the sets 111a, 111b alternatively or additionally comprise or consist of a metal or metal alloy, such as steel, which may provide higher thermal conductivity. In some embodiments, one of the abovementioned brake discs of the sets 111a, 111b may be of the plastic polymer material, and the other set of the metal.

As seen in Figs. 3 and 4, the control system 100 further comprises a helical compression spring 117a positioned between the base of the control wheel 101a and a spring holder 119a coaxially with the center shaft 103 of the control system 100 and the control wheel 101a. The spring 117a exerts a spring force on the stack 111 of brake discs 111a, 111b in the braking state of the multi-disc brake 110. The helical spring 117 is prestressed and is of steel. In other embodiments, any other suitable spring, e.g. a diaphragm spring, cup spring, disc spring, saucer spring, or leaf spring may be applied instead. The spring can alternatively be provided by one or more of the brake discs, e.g. the first brake disc 111b1, or one of the brake disc sets 111a, 111b, being of a resilient material, such as a resin or spring steel, and other discs or the other set of brake discs being of a substantially non-resilient material, such as non-spring steel. In other embodiments still, alternatively to a compression spring, the spring can be a tension spring or a drive spring in which case the action of the 105a and 106a may be reversed. The control system 100 can also include two or more springs for each brake 110a, 110b, which may be helical and/or compression springs, or a combination of the above spring types may be used. In other embodiments, instead of a spring, other means for activating the braking function of the stack of brake discs can be provided. Such other means may include an element, which can move a brake disc positioned at an end of the stack towards the other brake discs of the stack to provide the brake torque without providing a spring function. Such an element could be activated by a brake handle moving this element between braking and released states.

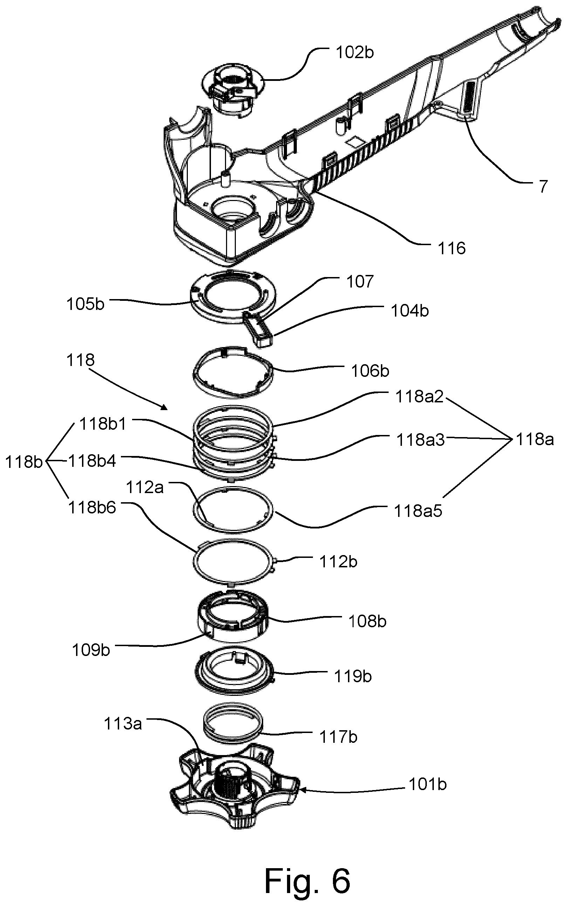

Referring especially to Fig. 6, the control system 100 comprises a further, second control wheel 101b connected to a further or second wire drum 102b for connection to a further steering wire of the endoscope 1, whereby rotation of the control wheel 101b controls the bending operation in another dimension than that of the control wheel 101a. The control system 100 also comprises a further, second multi-disc brake 110b comprising a stack 118, similarly with two brake disc sets 118a, 118b arranged similarly to the brake discs 111a, 111b of stack 111, wherein activation of the further multi-disc brake 110b similarly changes the multi-disc brake 110b from a released state to a braking state, a brake torque generated by the multi-disc brake 110b in the braking state braking rotation of the control wheel 101b, the brake torque in the released state being at least partially released. The control wheel 101b, wire drum 102b, associated steering wire (not shown), and multi-disc brake 110b are embodied as in the control wheel 101a, wire drum 102a, associated steering wire, and multi-disc brake 110a, respectively. Similar components and features of the further control wheel 101b have been given similar reference numbers as for the control wheel 101a described above, including spring 117b. For the control wheel 101b, instead of a control knob 104a, the activation of the multi-disc brake 110b is achieved by moving a brake handle 104b into an activation position. The brake handle 104b is positioned so that the brake handle 104b does not touch the control wheel 101b during the movement of the brake handle 104b. The brake handle 104b comprises an arm 107 that extends to the multi-disc brake 110b.

To guide and facilitate rotation of the wire drum 102b and control wheel 101b, the wire drum 102b and control wheel 101b are journaled in an outer bearing element 120b similar to the inner bearing element 120a described above. The outer bearing element is provided in one piece with the handle housing 116. In other embodiments, alternative suitable bearing devices may be applied.

The control wheel 101b is positioned coaxially with and axially shifted in relation to the control wheel 101a. A diameter of the control wheel 101b is larger than a diameter of the (first) control wheel 101a so that the outer one of the two control wheels 101a, 101b has a smaller diameter.

In the shown embodiment, the endoscope 1 is a gastroscope. In other embodiments, the endoscope 1 may be a duodenoscope or a colonoscope or any other type of endoscope.

In other embodiments, the control wheels, wire drums, and multi-disc brakes are embodied differently from each other.

In other embodiments, only a single control wheel and a single associated multi-disc brake is included in the control system.

### List of reference numerals:

- 1: Endoscope
- 2: Endoscope handle
- 3: Elongated insertion tube
- 3a: Proximal end of insertion tube
- 3b: Distal end of insertion tube
- 4: Tip
- 5: Bending section
- 6: Suction connector
- 7: Working channel port
- 100: Endoscope control system
- 101a: First control wheel
- 101b: Second control wheel
- 102a: First wire drum
- 102b: Second wire drum
- 103: Center shaft
- 104a: Brake knob
- 104b: Brake handle
- 105a: Rotation member
- 106a: Sliding member
- 106b: Sliding member
- 107: Arm
- 108a: Disc holder
- 108b: Disc holder
- 109a: Disc holder grooves
- 109b: Disc holder grooves
- 110a: First multi-disc brake
- 110b: Second multi-disc brake
- 111: Stack of brake discs
- 111a: Set of inner brake discs
- 111a2: Inner brake disc
- 111a3: Inner brake disc
- 111a4: Inner brake disc
- 111b: Set of outer brake discs
- 111b1: Outer brake disc
- 111b4: Outer brake disc
- 111b6: Outer brake disc
- 112a: Inner brake disc protrusion
- 112b: Outer brake disc protrusion
- 113a: Control wheel grooves
- 114: Center shaft grooves
- 115: Frame
- 116: Handle housing
- 117a: Spring
- 117b: Spring
- 118: Stack of brake discs
- 118a: Set of inner brake discs
- 118b: Set of outer brake discs
- 119a: Spring holder
- 119b: Spring holder
- 120a: Inner bearing element
- 120b: Outer bearing element

## Claims

1. An endoscope control system (100) for performing a bending operation in a disposable insertion endoscope, the endoscope control system comprising:
a control wheel (101a; 101b) connected to a wire drum (102a; 102b) for connection to a steering wire of the endoscope, whereby rotation of the control wheel controls the bending operation; and
a multi-disc brake (110a; 110b) comprising a stack of at least three brake discs (111a, 111b; 118a, 118b), wherein activation of the multi-disc brake (110a; 110b) changes the multi-disc brake (110a; 110b) from a released state to a braking state, a brake torque generated by the multi-disc brake (110a; 110b) in the braking state braking rotation of the control wheel (101a; 101b), the brake torque in the released state being at least partially released, wherein the control system further comprises a spring (117a; 117b) exerting a spring force on the stack of brake discs (111a, 111b; 118a, 118b) in an engaged state of the multi-disc brake (110a; 110b) as a means for activating the braking function of the stack of brake discs (111a, 111b; 118a, 118b), **characterised in that** the stack of brake discs (111a, 111b; 118a, 118b) consists of four or more discs.

2. The control system according to claim 1, wherein the spring is pre-compressed and is compressed further when engaging the brake.

3. The control system according to claim 1 or 2, wherein the stack includes a first brake disc (111b1; 118b1) positioned between a second and a third brake disc (111a2, 111a3; 118a2, 118a3) of the stack, the first brake disc (111b1; 118b1) being rotatable in relation to the second and third brake discs (111a2, 111a3; 118a2, 118a3), a first friction interface being provided between the first and second discs (111b1, 111a2; 118b1, 118a2) and a second friction interface being provided between the first and third brake discs (111b1, 111a3; 118b1, 118a3), so that rotation of the first brake disc (111b1; 118b1) in relation to the second and third brake discs (111a2, 111a3; 118a2, 118a3) activates the first and second friction interfaces to provide at least part of the brake torque.

4. The control system according to claim 3, wherein, when moving the multi disc brake (110a; 110b) from the released state to the braking state, a force is exerted on the stack of discs (111a, 111b; 118a, 118b), the force pushing the second and third discs (111a2, 111a3; 118a2, 118a3) towards the first disc (111b1; 118b1), the first and second friction interfaces thereby providing a brake force against rotation of the first brake disc (111b1; 118b1) relative to the second and third brake discs (111a2, 111a3; 118a2, 118a3).

5. The control system according to claim 3 or 4, further comprising a frame (115), the rotation of the control wheel (101a; 101b) occurring relative to the frame (115), and wherein the first brake disc (111b1; 118b1) is rotationally fixed relative to the control wheel (101a; 101b), and the second and third brake discs (111a2, 111a3; 118a2, 118a3) are rotationally fixed relative to the frame (115).

6. The control system according to claim 5, wherein the frame (115) is fixed to or forms part of an endoscope handle housing (116) of an endoscope handle (2).

7. The control system according to any one of the previous claims, wherein the stack consists of six brake discs (111a1, 111a2, 111a3, 111b1, 111b2, 111b3; 118a1, 118a2, 118a3, 118b1, 118b2, 118b3).

8. The control system according to any one of the previous claims, wherein every other of the brake discs of the stack (111a, 111b; 118a, 118b) is manufactured from a material different from a material of the remaining brake discs.

9. The control system according to any one of the previous claims, wherein at least one of the brake discs (111a1, 111a2, 111a3, 111b1, 111b2, 111b3; 118a1, 118a2, 118a3, 118b1, 118b2, 118b3) is manufactured from plastic polymer material.

10. The control system according to any one of the previous claims, including a brake handle (104a; 104b), wherein movement of the brake handle (104a; 104b) is transferred to the multi-disc brake by the brake handle (104a; 104b) rotating a rotation member (105a; 105b) relative to a sliding member (106a; 106b), the sliding member (106a; 106b) providing a pushing force on at least one of the brake discs (111a2; 118a2) activating the braking torque on the multi-disc brake.

11. The control system according to any one of the previous claims, further comprising
a further control wheel (101b; 101a) connected to a further wire drum for connection to a further steering wire of the endoscope, whereby rotation of the further control wheel controls the bending operation in another dimension than that of the control wheel; and
a further multi-disc brake (110b; 110a) comprising a stack of at least three brake discs, wherein activation of the further multi-disc brake changes the further multi-disc brake from a released state to a braking state, a brake torque generated by the further multi-disc brake in the braking state braking rotation of the further control wheel, the brake torque in the released state being at least partially released.

12. An endoscope handle (2) for an endoscope (1), the endoscope handle (2) comprising a control system (100) according to any one of the previous claims.

13. An endoscope (1) comprising a control system (100) according to any one of claims 1 to 11 and/or comprising an endoscope handle (2) according to claim 12.

14. The endoscope (1) according to claim 13, further comprising a distal tip (4) or tip part that comprises a bending section (5) connected to the steering wire(s) so that the control system (100) can activate a bending operation of the bending section (5) via the steering wire(s).

## Patentansprüche

1. Endoskopsteuersystem (100) zur Durchführung eines Biegevorgangs in einem Einweg-Einführendoskop, wobei das Endoskopsteuersystem umfasst:
ein Stellrad (101a; 101b), das mit einer Drahttrommel (102a; 102b) zur Verbindung mit einem Führungsdraht des Endoskops verbunden ist, wobei Drehung des Stellrads den Biegevorgang steuert; und
eine Lamellenbremse (110a; 110b), die einen Stapel von wenigstens drei Bremsscheiben (111a, 111b; 118a, 118b) umfasst, wobei Aktivierung der Lamellenbremse (110a; 110b) die Lamellenbremse (110a; 110b) von einem gelösten Zustand in einen Bremszustand ändert, wobei ein durch die Lamellenbremse (110a; 110b) in dem Bremszustand erzeugtes Bremsmoment Drehung des Stellrads (101a; 101b) bremst, wobei das Bremsmoment in dem gelösten Zustand wenigstens teilweise gelöst ist, wobei das Steuersystem ferner eine Feder (117a; 117b) umfasst, die eine Federkraft auf den Stapel von Bremsscheiben (111a, 111b; 118a, 118 111b) in einem Eingriffszustand der Lamellenbremse (110a; 110b) ausübt, als Mittel zum Aktivieren der Bremsfunktion des Stapels von Bremsscheiben (111a, 111b; 118a, 118b), **dadurch gekennzeichnet, dass** der Stapel von Bremsscheiben (111a, 118a, 118b) aus vier oder mehr Scheiben besteht.

2. Steuersystem nach Anspruch 1, wobei die Feder vorkomprimiert ist und bei Eingriff der Bremse weiter komprimiert wird.

3. Steuersystem nach Anspruch 1 oder 2, wobei der Stapel eine erste Bremsscheibe (111b1; 118b1) umfasst, die zwischen einer zweiten und einer dritten Bremsscheibe (111a2, 111a3; 118a2, 118a3) des Stapels angeordnet ist, wobei die erste Bremsscheibe (111b1; 118b1) bezogen auf die zweite und dritte Bremsscheibe (111a2, 111a3; 118a2, 118a3) drehbar ist, wobei eine erste Reibfläche zwischen der ersten und der zweiten Scheibe (111b1, 111a2; 118b1, 118a2) bereitgestellt ist und eine zweite Reibfläche zwischen der ersten und dritten Bremsscheibe (111b1, 111a3; 118b1, 118a3) bereitgestellt ist, so dass Drehung der ersten Bremsscheibe (111b1; 118b1) relativ zu der zweiten und dritten Bremsscheibe (111a2, 111a3; 118a2, 118a3) die erste und zweite Reibfläche aktiviert, um wenigstens einen Teil des Bremsmoments bereitzustellen.

4. Steuersystem nach Anspruch 3, wobei bei Bewegen der Lamellenbremse (110a; 110b) aus dem gelösten Zustand in den Bremszustand eine Kraft auf den Scheibenstapel (111a, 111b; 118a, 118b) ausgeübt wird, wobei die Kraft die zweite und die dritte Scheibe (111a2, 111a3; 118a2, 118a3) in Richtung zu der ersten Scheibe (111b1; 118b1) drückt, wodurch die erste und die zweite Reiffläche ein Bremskraft gegen Drehung der ersten Bremsscheibe (111b1; 118b1) relativ zu der zweiten und dritten Bremsscheibe (111a2, 111a3; 118a2, 118a3) bereitstellt.

5. Steuersystem nach Anspruch 3 oder 4, ferner umfassend einen Rahmen (115), wobei die Drehung des Stellrads (101a; 101b) relativ zu dem Rahmen (115) erfolgt und wobei die erste Bremsscheibe (111b1; 118b1) relativ zu dem Stellrad (101a; 101b) drehfest ist und die zweite und dritte Bremsscheibe (111a2, 111a3; 118a2, 118a3) relativ zu dem Rahmen (115) drehfest sind.

6. Steuersystem nach Anspruch 5, wobei der Rahmen (115) an einem Endoskopgriffgehäuse (116) eines Endoskopgriffes (2) befestigt ist oder einen Teil davon bildet.

7. Steuersystem nach einem der vorstehenden Ansprüche, wobei der Stapel aus sechs Bremsscheiben (111a1, 111a2, 111a3, 111b1, 111b2, 111b3; 118a1, 118a2, 118a3, 118b1, 118b2, 118b3) besteht.

8. Steuersystem nach einem der vorstehenden Ansprüche, wobei jede zweite der Bremsscheiben des Stapels (111a, 111b; 118a, 118b) aus einem Material hergestellt ist, das sich von einem Material der restlichen Bremsscheiben unterscheidet.

9. Steuersystem nach einem der vorstehenden Ansprüche, wobei wenigstens eine der Bremsscheiben (111a1, 111a2, 111a3, 111b1, 111b2, 111b3; 118a1, 118a2, 118a3, 118b1, 118b2, 118b3) aus Kunststoff-Polymermaterial hergestellt ist.

10. Steuersystem nach einem der vorstehenden Ansprüche, das einen Bremsgriff (104a; 104b) aufweist, wobei Bewegung des Bremsgriffs (104a; 104b) auf die Lamellenbremse übertragen wird, indem der Bremsgriff (104a; 104b) ein Rotationselement (105a; 105b) relativ zu einem Gleitelement (106a; 106b) dreht, wobei das Gleitelement (106a; 106b) eine Druckkraft auf wenigstens eine der Bremsscheiben (111a2; 118a2) bereitstellt, um das Bremsmoment der Lamellenbremse zu aktivieren.

11. Steuersystem nach einem der vorstehenden Ansprüche, ferner umfassend
ein weiteres Stellrad (101b; 101a), das mit einer weiteren Drahttrommel zur Verbindung mit einem weiteren Führungsdraht des Endoskops verbunden ist, wobei Drehung des weiteren Stellrads den Biegevorgang in einer anderen Dimension als jene des Stellrads steuert; und
eine weitere Lamellenbremse (110b; 110a), die einen Stapel von wenigstens drei Bremsscheiben umfasst, wobei Aktivierung der weiteren Lamellenbremse die weitere Lamellenbremse von einem gelösten Zustand in einen Bremszustand ändert, wobei ein von der weiteren Lamellenbremse im Bremszustand erzeugtes Bremsmoment Drehung des weiteren Stellrads bremst, wobei das Bremsmoment in dem gelösten Zustand wenigstens teilweise gelöst ist.

12. Endoskopgriff (2) für ein Endoskop (1), wobei der Endoskopgriff (2) ein Steuersystem (100) nach einem der vorstehenden Ansprüche umfasst.

13. Endoskop (1) umfassend ein Steuersystem (100) nach einem der Ansprüche 1 bis 11 und/oder umfassend einen Endoskopgriff (2) nach Anspruch 12.

14. Endoskop (1) nach Anspruch 13, ferner umfassend eine distale Spitze (4) oder einen distalen Spitzenteil, der/die einen Biegeabschnitt (5) umfasst, der mit dem/den Führungsdrähten verbunden ist, so dass das Steuersystem (100) einen Biegevorgang des Biegeabschnitts (5) über den/die Führungsdrähte aktivieren kann.

## Revendications

1. Système de commande d'endoscope (100) pour effectuer une opération de flexion dans un endoscope jetable à insertion, le système de commande d'endoscope comprenant :
une roue de commande (101a ; 101b) reliée à un tambour de fil (102a ; 102b) pour être reliée à un fil d'orientation de l'endoscope, la rotation de la roue de commande commandant l'opération de flexion ; et
un frein multidisque (110a ; 110b) comprenant un empilement d'au moins trois disques de frein (111a, 111b ; 118a, 118b), l'activation du frein multidisque (110a ; 110b) faisant passer le frein multidisque (110a ; 110b) d'un état relâché à un état de freinage, un couple de freinage étant généré par le frein multidisque (110a ; 110b) dans l'état de freinage freinant la rotation de la roue de commande (101a ; 101b), le couple de freinage à l'état relâché étant au moins partiellement relâché, le système de commande comprenant en outre un ressort (117a ; 117b) exerçant une force de ressort sur l'empilement de disques de frein (111a, 111b ; 118a, 118b) dans un état enclenché du frein multidisque (110a ; 110b) en tant que moyen pour activer la fonction de freinage de l'empilement de disques de frein (111a, 111b ; 118a, 118b), **caractérisé en ce que** l'empilement de disques de frein (111a, 111b ; 118a, 118b) se compose de quatre disques ou plus.

2. Système de commande selon la revendication 1, le ressort étant pré-comprimé et étant comprimé davantage lors de l'engagement du frein.

3. Système de commande selon la revendication 1 ou 2, l'empilement comprenant un premier disque de frein (111b1 ; 118b1) positionné entre un deuxième et un troisième disque de frein (111a2, 111a3 ; 118a2, 118a3) de l'empilement, le premier disque de frein (111b1 ; 118b1) pouvant tourner par rapport aux deuxième et troisième disques de frein (111a2, 111a3 ; 118a2, 118a3), une première interface de friction étant prévue entre les premier et deuxième disques (111b1, 111a2 ; 118b1, 118a2) et une deuxième interface de friction étant prévue entre les premier et troisième disques de frein (111b1, 111a3 ; 118b1, 118a3), de sorte que la rotation du premier disque de frein (111b1 ; 118b1) par rapport aux deuxième et troisième disques de frein (111a2, 111a3 ; 118a2, 118a3) active les première et deuxième interfaces de friction pour fournir au moins une partie du couple de freinage.

4. Système de commande selon la revendication 3, lors du déplacement du frein multidisque (110a ; 110b) de l'état relâché à l'état de freinage, une force étant exercée sur l'empilement de disques (111a, 111b ; 118a, 118b), la force poussant les deuxième et troisième disques (111a2, 111a3 ; 118a2, 118a3) vers le premier disque (111b1 ; 118b1), les première et deuxième interfaces de friction fournissant ainsi une force de freinage contre la rotation du premier disque de frein (111b1 ; 118b1) par rapport aux deuxième et troisième disques de frein (111a2, 111a3 ; 118a2, 118a3).

5. Système de commande selon la revendication 3 ou 4, comprenant en outre un châssis (115), la rotation de la roue de commande (101a ; 101b) se produisant par rapport au châssis (115), et le premier disque de frein (111b1 ; 118b1) étant fixe en rotation par rapport à la roue de commande (101a ; 101b), et les deuxième et troisième disques de frein (111a2, 111a3 ; 118a2, 118a3) étant fixes en rotation par rapport au châssis (115).

6. Système de commande selon la revendication 5, le châssis (115) étant fixé à ou faisant partie d'un logement de poignée d'endoscope (116) d'une poignée d'endoscope (2).

7. Système de commande selon l'une quelconque des revendications précédentes, l'empilement se composant de six disques de frein (111a1, 111a2, 111a3, 111b1, 111b2, 111b3 ; 118a1, 118a2, 118a3, 118b1, 118b2, 118b3).

8. Système de commande selon l'une quelconque des revendications précédentes, un disque de frein sur deux de l'empilement (111a, 111b ; 118a, 118b) étant fabriqué à partir d'un matériau différent de celui des disques de frein restants.

9. Système de commande selon l'une quelconque des revendications précédentes, au moins l'un des disques de frein (111a1, 111a2, 111a3, 111b1, 111b2, 111b3 ; 118a1, 118a2, 118a3, 118b1, 118b2, 118b3) étant fabriqué à partir d'un matériau polymère plastique.

10. Système de commande selon l'une quelconque des revendications précédentes, comprenant une poignée de frein (104a ; 104b), le mouvement de la poignée de frein (104a ; 104b) étant transféré au frein multidisque par la poignée de frein (104a ; 104b) faisant tourner un élément de rotation (105a ; 105b) par rapport à un élément coulissant (106a ; 106b), l'élément coulissant (106a ; 106b) fournissant une force de poussée sur au moins l'un des disques de frein (111a2 ; 118a2), activant le couple de freinage sur le frein multidisque.

11. Système de commande selon l'une quelconque des revendications précédentes, comprenant en outre :
une roue de commande supplémentaire (101b ; 101a) reliée à un tambour de fil supplémentaire pour être reliée à un fil d'orientation supplémentaire de l'endoscope, la rotation de la roue de commande supplémentaire commandant l'opération de flexion dans une autre dimension que celle de la roue de commande ; et
un frein multidisque supplémentaire (110b ; 110a) comprenant un empilement d'au moins trois disques de frein, l'activation du frein multidisque supplémentaire faisant passer le frein multidisque supplémentaire d'un état relâché à un état de freinage, un couple de freinage généré par le frein multidisque supplémentaire dans l'état de freinage en freinant la rotation de l'autre roue de commande, le couple de freinage dans l'état relâché étant au moins partiellement relâché.

12. Poignée d'endoscope (2) pour un endoscope (1), la poignée d'endoscope (2) comprenant un système de commande (100) selon l'une quelconque des revendications précédentes.

13. Endoscope (1) comprenant un système de commande (100) selon l'une quelconque des revendications 1 à 11 et/ou comprenant une poignée d'endoscope (2) selon la revendication 12.

14. Endoscope (1) selon la revendication 13, comprenant en outre une pointe distale (4) ou partie de pointe qui comprend une section de flexion (5) reliée au ou aux fils d'orientation de sorte que le système de commande (100) puisse activer une opération de flexion de la section de flexion (5) par l'intermédiaire des fils d'orientation.
